# EUROPEAN PATENT APPLICATION

(11) **EP 4 650 368 A1**
(43) Date of publication of application: **19.11.2025**
(21) Application number: 24175368.0
(22) Date of filing: 13.05.2024
(51) Int. Cl.: C07K 16/28, A61P 7/06, A61P 13/12

(54) **TETRAVALENT AGONISTS OF ERYTHROPOIETIN RECEPTOR**

(71) Applicant: Epok Therapeutics Inc., Toronto, ON M5H 2T6 (CA)
(72) Inventor: BLAZER, Levi L., Toronto M5H 2T6 Ontario (CA); ADAMS, Jarrett, Toronto M5H 2T6 Ontario (CA); MOE, Orson W., Toronto M5H 2T6 Ontario (CA); SIDHU, Sachdev, Toronto M5H 2T6 Ontario (CA)
(74) Representative: Rigamonti, Dorotea

(57) **Abstract**

It forms an object of the present invention a tetravalent antibody construct capable of selectively binding with high affinity to human EPOR, acting as as hEPOR agonists.

It forms a further object said antibodies for use in the treatment of kidney disease and anaemias.

## Description

The present invention relates to EPOR (Erythropoietin Receptor) agonists and antagonists in the diagnosis and treatment of disease, particularly kidney disease and anaemias arising from cancer treatment, or inherited syndromes. More particularly, the present invention is directed to synthetic EPOR tetravalent antibody constructs. These EPOR agonists and antagonists represent a novel therapeutic option for patients with chronic kidney disease, treatment-related anaemia, and inherited erythropoietin deficiency.

### BACKGROUND

Human erythropoietin (hEPO) is a growth factor that promotes red blood cell generation. It binds to the human EPO receptor (hEPOR) on the red blood cell progenitors and activates an intracellular signalling cascade involved in erythropoiesis. Naturally, it is produced by the peritubular cells of the kidney in response to hypoxia. Patients with chronic kidney disease (CKD) very often have reduced EPO production and consequently suffer from anaemia, a condition of red blood cell deficiency. As a result, patients require frequent administration of recombinant EPO or other Erythropoiesis-Stimulating Agents (ESAs) to maintain normal red blood cell production. The most common ESAs are epoetin alfa and darbepoetin alfa, which are given to patients at a dosing interval of 2 to 3 times weekly and every 1 to 2 weeks, respectively.

Erythropoiesis begins with lineage commitment of Hematopoietic Stem and Progenitor Cells (HSPCs) into the erythroid lineage. As HSPCs differentiate into erythroid progenitors, they begin to express EPOR and become responsive to circulating EPO that promotes further proliferation and eventual maturation into differentiated red blood cells. The binding of hEPO to hEPOR occurs via high- and low-affinity binding sites (Sites 1 and 2, respectively), inducing conformational changes in the receptor, followed by the activation of downstream signalling cascades. Inactive hEPOR is thought to be expressed at the cell surface as a pre-formed dimer in the absence of ligand. hEPO binding proceeds by an asymmetrical association, first via the high affinity hEPO Site 1 followed by the lower affinity Site 2 interaction, which stimulates a reorientation of hEPOR monomers within the dimeric complex and subsequent activation of the JAK2 receptor-associated kinase.

In addition to CKD, anaemia is a common complication that affects ~40% of cancer patients and ~90% of patients receiving chemotherapy. While recombinant hEPO is an effective treatment for cancer-associated anaemia, its adverse effects on the disease recurrence and patient survival have raised concerns and precluded its use in many oncology patients. hEPO has deleterious effects on cancer patients, at least in part, through non-erythropoietic signalling pathways such as Ephrin B4 receptor (EPHB4) and IL-3R common β-subunit (CD131). hEPO may also increase cancer growth through increased oxygen delivery to hypoxic areas within the tumour. The pleiotropic effects of EPO highlight the need and the opportunity not only for the development of EPOR-stimulating, erythropoiesis-specific therapies but also ways to selectively inhibit EPO signalling mechanisms that promote cancer.

There remains to be identified agonists and antagonists of EPOR that meet the challenges noted above that aid in the diagnosis and treatment of diseases, particularly anaemias of cancer and other disease conditions.

### SUMMARY OF THE INVENTION

The present invention provides **tetravalent antibody constructs** capable of selectively binding with high affinity to human EPOR. Said constructs can act as hEPOR agonists.

The authors of the present invention demonstrated that an optimal agonist activity on hEPOR is obtained with tetravalent antibody constructs, wherein the structure of said antibodies drastically impacts the effect, as it will be clear from the experimental data that follow. In a preferred embodiment, said tetravalent antibody construct is a tetrabody.

An embodiment of the present invention is the **use** of said hEPOR agonists as diagnostic agents and therapeutic agents for the treatment of disease, including CKD, cancer-associated or treatment-related anaemias and inherited anaemias.

According to an embodiment, the tetravalent antibody constructs comprise a CDR-L1 having a contiguous amino acid sequence SVSSA (SEQ ID NO: 1).

According to another embodiment, the constructs further comprise a CDR-L2 having a contiguous amino acid sequence SASSLYS (SEQ ID NO: 2).

According to another embodiment, the constructs further comprise a CDR-L3 having a contiguous amino acid sequence SSXFLI (SEQ ID NO: 3). In an embodiment, X is N or Q.

According to another embodiment, the constructs further comprise a CDR-H1 having a contiguous amino acid sequence LDSYYM (SEQ ID NO: 4).

According to another embodiment, the constructs further comprise a CDR-H2 having a contiguous amino acid sequence SIAPYHGYTY (SEQ ID NO: 5).

According to another embodiment, the constructs further comprise a CDR-H3 having a contiguous amino acid sequence HGYGAL (SEQ ID NO: 6).

According to one embodiment, there is provided a tetravalent antibody construct comprising: (a) a CDR-L1 as described herein; (b) a CDR-L2 as described herein; and (c) a CDR-L3 as described herein. According to one embodiment, a tetravalent antibody construct of this invention comprises: (a) a CDR-H1 as described herein; (b) a CDR-H2 as described herein; and (c) a CDR-H3 as described herein. According to yet another embodiment of the present invention, there is provided a tetravalent antibody construct comprising: (a) a CDR-L1 as described herein; (b) a CDR-L2 as described herein; (c) a CDR-L3 as described herein; (d) a CDR-H1 as described herein; (e) a CDR-H2 as described herein; and (e) a CDR-H3 as described herein.

Various forms of the antibody, diabodies, other binding molecules and variants thereof are contemplated herein. For example, the antibody may be a full-length antibody (e.g., having a human immunoglobulin constant region) or an antibody fragment (e.g., a Fab or F(ab')2) or a diabody. Furthermore, the constructs of the present invention may be labelled with a detectable label, immobilized on a solid phase and/or conjugated with a heterologous compound (such as a cytotoxic agent).

Diagnostic and therapeutic uses for the constructs of the present invention are contemplated. In one diagnostic application, the invention provides a method for determining the presence of a protein of interest comprising exposing a sample suspected of containing the protein to the construct of the present invention and determining binding of the construct to the sample. For this use, the invention may include a kit comprising a construct of the present invention and instructions for using it to detect the protein.

The invention also provides a composition comprising at least one construct of the present invention and pharmaceutically acceptable carriers or diluents. This composition for therapeutic use is sterile and may be lyophilized. Also contemplated is the use of a construct of this invention in the manufacture of a medicament for treating an indication described herein.

The invention further provides a method for treating a mammal, comprising administering an effective amount of the embodiments of the present invention to the mammal. The mammal to be treated in the method may be a nonhuman mammal, e.g., a primate suitable for gathering preclinical data or a rodent (e.g., mouse or rat or rabbit). The nonhuman mammal may be healthy (e.g., in toxicology studies) or may be suffering from a disorder to be treated with the composition or compound of interest. In one embodiment, the mammal is suffering from or is at risk of developing a disorder. In one specific embodiment, the disorder is kidney disease, cancer-associated or treatment-related anaemias, and inherited anaemia syndromes. The amount of composition or construct administered will be a therapeutically effective amount to treat the disorder. In dose escalation studies, a variety of doses of the construct or composition may be administered to the mammal. In another embodiment, a therapeutically effective amount of the composition or construct is administered to a human patient to treat a disorder in that patient. Accordingly, the constructs of the present invention can be used in the manufacture of a medicament for treating kidney disease, cancer-associated or treatment-related anaemias, and inherited anaemias.

### BRIEF DESCRIPTION OF THE DRAWINGS

The foregoing and other objects, features and advantages of the present invention should become apparent from the following description when taken in conjunction with the accompanying drawings.

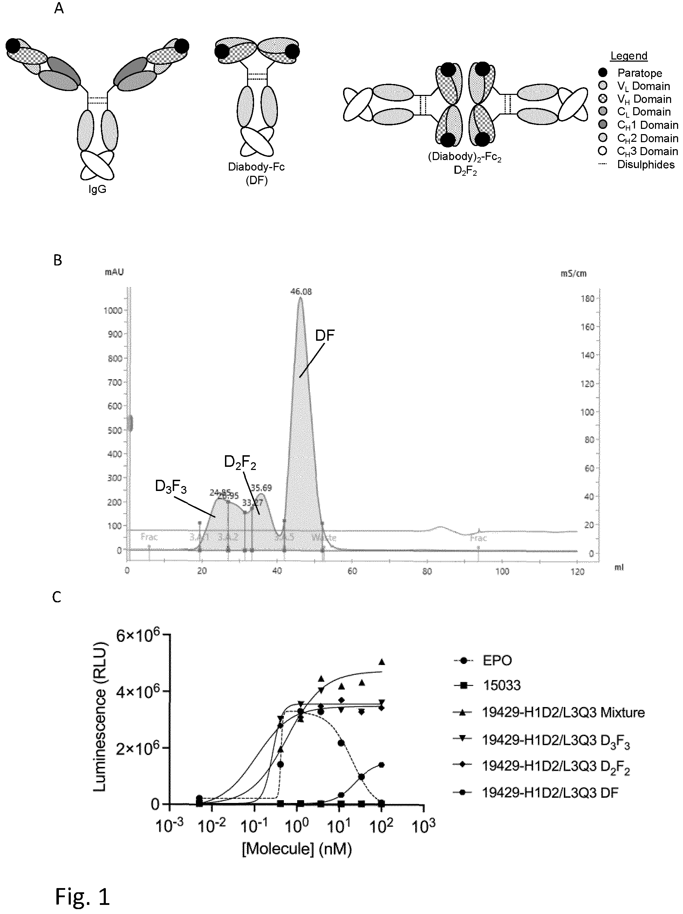

**FIGS. 1A to 1C** show the basic IgG and DF constructs. **A.** A schematic showing different formats of bivalent (IgG, DF) (comparative) and tetravalent constructs (Diabody₂-Fc₂, D₂F₂) showing the variable heavy and light chains, the linkages, and constant regions. **B.** Size Exclusion Chromatography (SEC) performed on DF. **C.** Peaks isolated from the SEC were tested for their ability to induce human erythroid UT7 cell proliferation. EPO and 15033 DF were used as positive and negative controls, respectively.

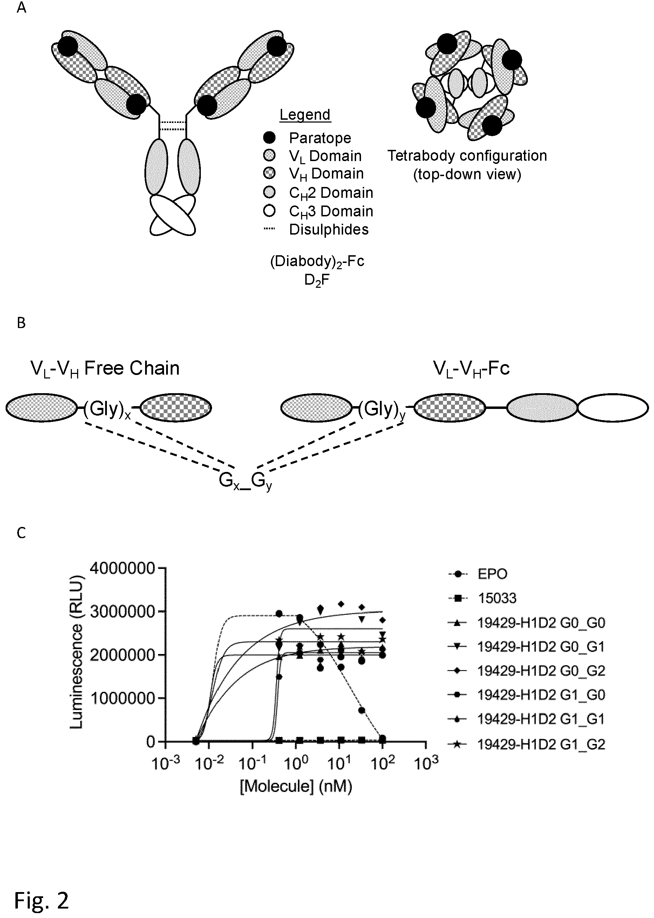

**FIGS. 2A to 2F** show the structures and activity of the D₂F construct. **A.** Representative schematics showing an embodiment of the tetravalent construct D₂F with the variable heavy and light chains, the disulphide bonds, and constant regions. The left panel shows the free VL-VH chain non-covalently associating with a VL-VH-Fc chain. The right panel shows a second active configuration of the D₂F format in which a tetrabody is formed (Todorovskov A et al. Design and application of diabodies, triabodies, and tetrabodies for cancer targeting. J Immunol Methods. 2001; 248:47-66). **B.** A schematic showing the linkers in D₂F connecting the variable heavy and light chains. **C.** Differently linked constructs were tested for their ability to induce UT7 cell proliferation compared to EPO. **D.** SEC performed on the best-in-class (i.e. most homogeneous) construct, G1_G1. **E.** Peaks isolated from the SEC were tested for their ability to induce UT7 cell proliferation compared to EPO. **F.** SDS-PAGE analysis under non-reducing and reducing conditions of the G1_G1 D₂F molecule.

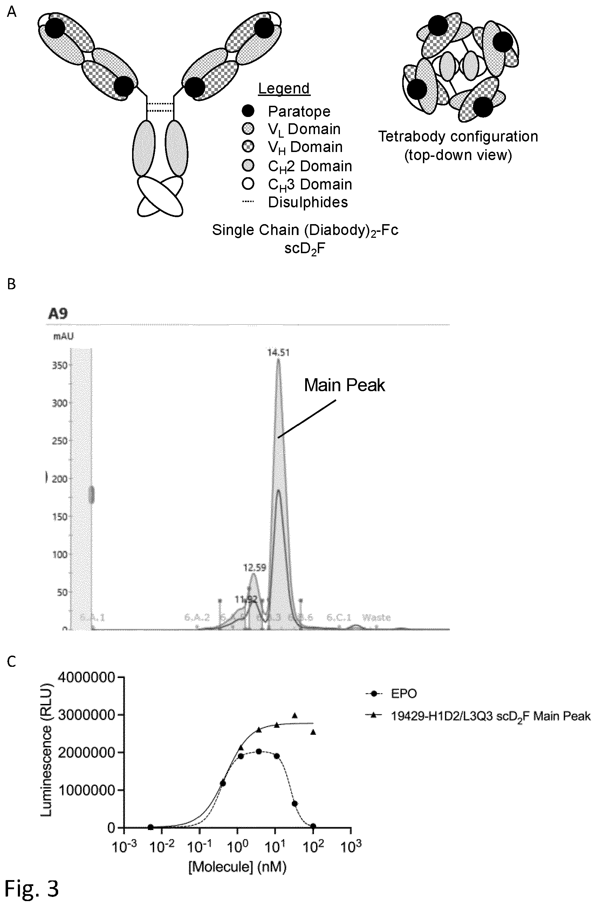

**FIGS. 3A to 3C** show the structures and activity of the single chain D₂F (scD₂F) construct. **A.** Representative schematics showing an embodiment of the tetravalent construct scD₂F with the variable heavy and light chains, the disulphide bonds and constant regions. The left panel shows a configuration where each single chain folds back onto itself. The right panel shows a second alternative configuration where a tetrabody is formed consisting of a lattice of the variable domains (Todorovskov A et al. Design and application of diabodies, triabodies, and tetrabodies for cancer targeting. J Immunol Methods. 2001; 248:47-66). **B.** SEC performed on scD₂F. C. Peaks isolated from the SEC were tested for their ability to induce UT7 cell proliferation compared to EPO.

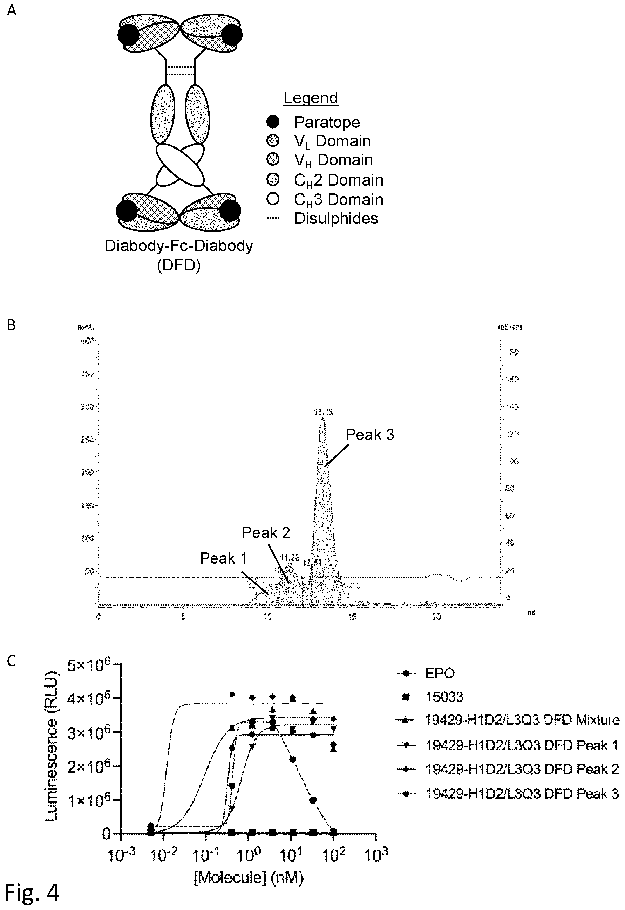

**FIGS. 4A to 4C** show the structure and activity of the DFD construct. **A.** A representative schematic showing an embodiment of the tetravalent construct DFD with the variable heavy and light chains, the disulphide bonds, and constant regions. **B.** SEC performed on DFD. C. Peaks isolated from the SEC were tested for their ability to induce UT7 cell proliferation compared to EPO.

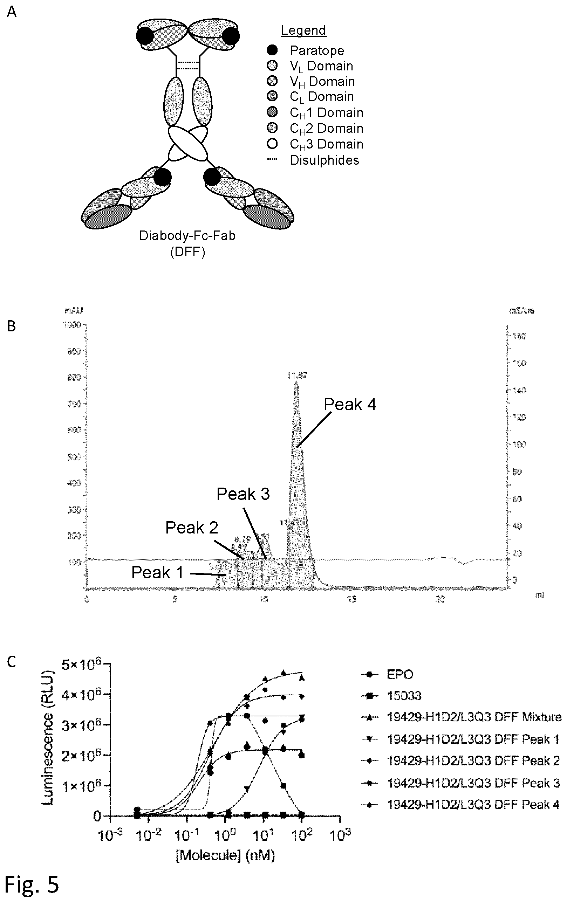

**FIGS. 5A to 5C** show the structure and activity of the DFF construct. **A.** A representative schematic showing an embodiment of the tetravalent construct DFF with the variable heavy and light chains, the disulphide bonds, and constant regions. **B.** SEC performed on DFF. **C.** Peaks isolated from the SEC were tested for their ability to induce UT7 cell proliferation compared to EPO.

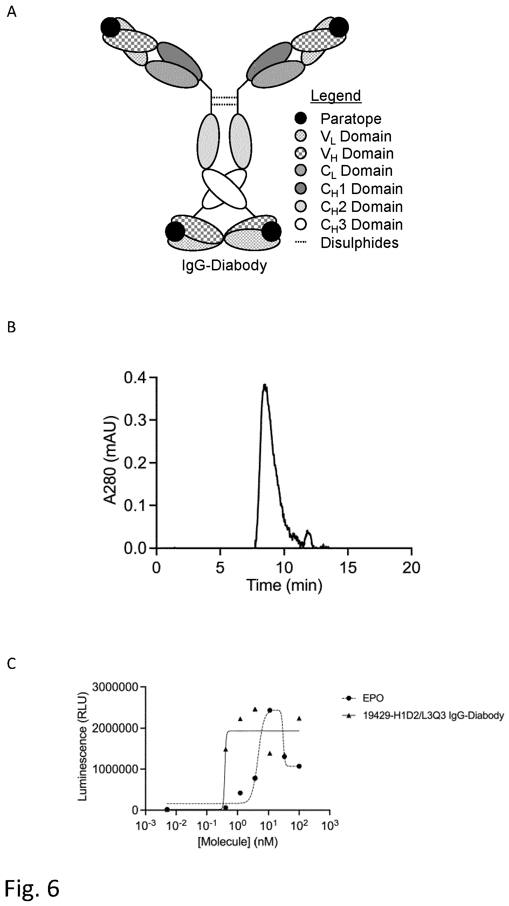

**FIG. 6A to 6C** show the structure and activity of the IgG-D construct. **A.** A representative schematic showing an embodiment of the tetravalent construct IgG-D with the variable heavy and light chains, the disulphide bonds, and constant regions. **B.** SEC performed on IgG-D. **C.** IgG-D ability to induce U17 cell proliferation compared to EPO.

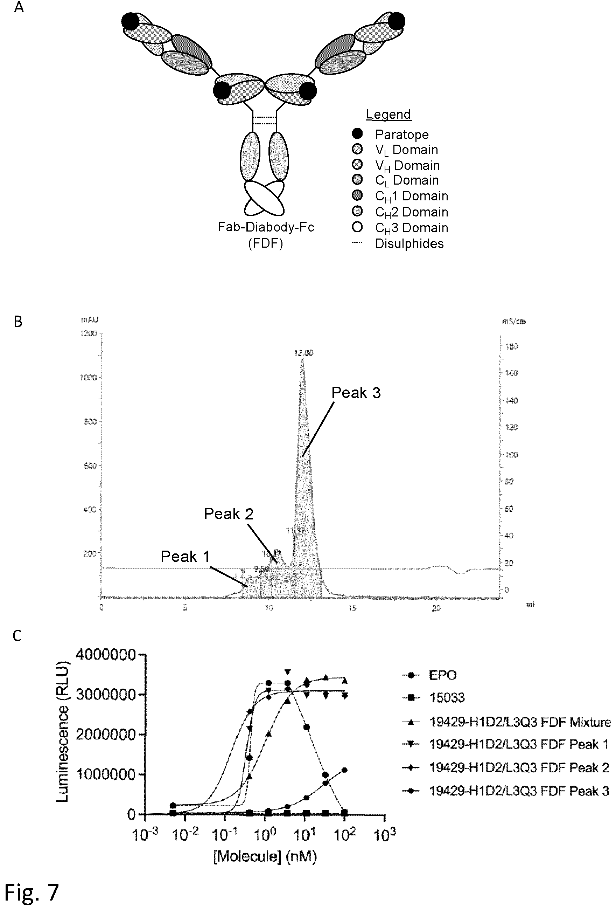

**FIG. 7A to 7C** show the structure and activity of the FDF construct. **A.** A representative schematic showing an embodiment of the tetravalent construct FDF showing the variable heavy and light chains, the disulphides bond, and constant regions. **B.** SEC performed on FDF. C. Peaks isolated from the SEC were tested for their ability to induce UT7 cell proliferation compared to EPO.

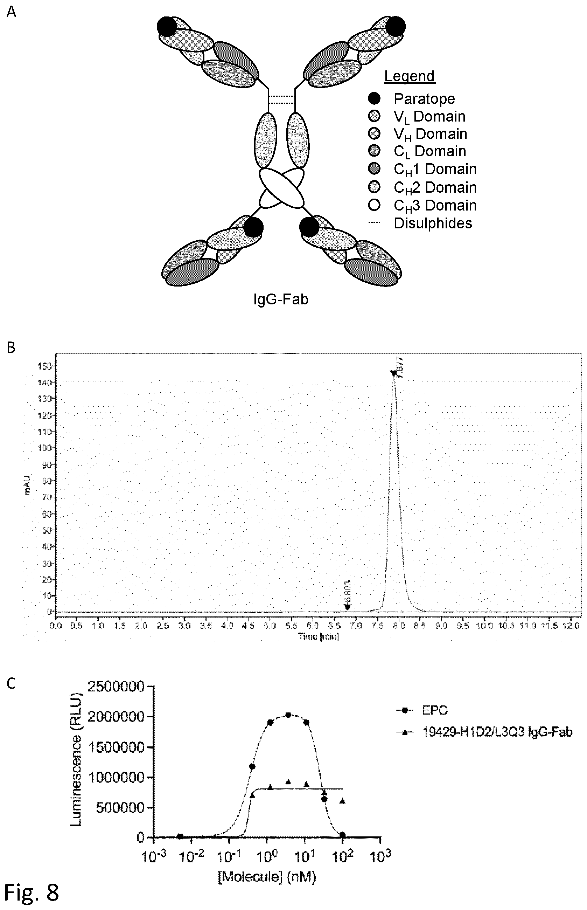

**FIGS. 8A to 8C** show the structure and activity of the IgG-Fab construct. **A.** A representative schematic showing an embodiment of the tetravalent construct IgG-Fab with the variable heavy and light chains, the disulphide bonds, and constant regions. **B.** SEC performed on IgG-Fab, showing a single peak. **C.** The single peak was tested for its ability to induce UT7 cell proliferation compared to EPO.

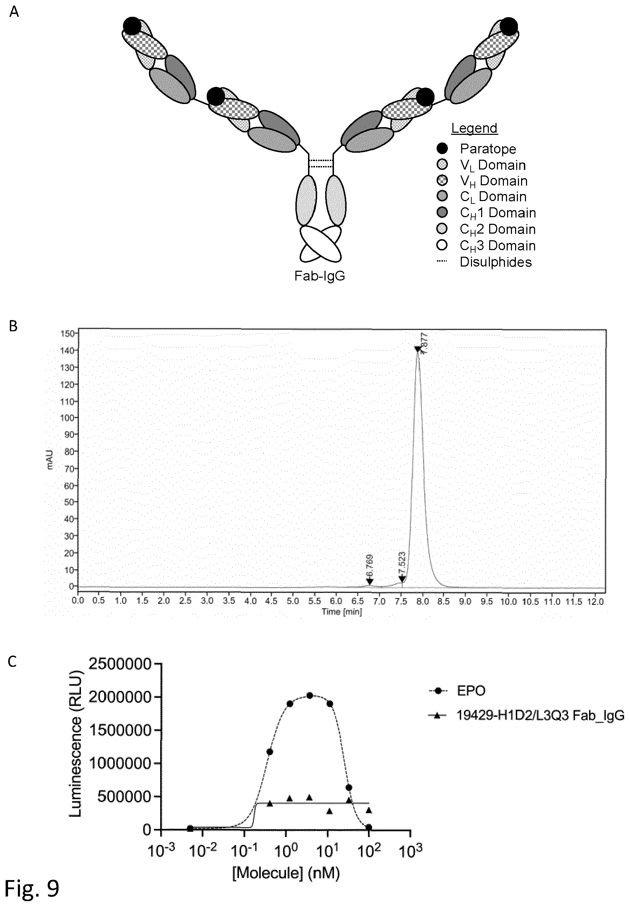

**FIGS. 9A to 9C** show the structure and activity of the Fab-IgG construct. **A.** A representative schematic showing the tetravalent construct Fab-IgG with the variable heavy and light chains, the disulphide bonds, and constant regions. **B.** SEC performed on Fab-IgG, showing a single peak. C. The single peak was tested for its ability to induce UT7 cell proliferation compared to EPO.

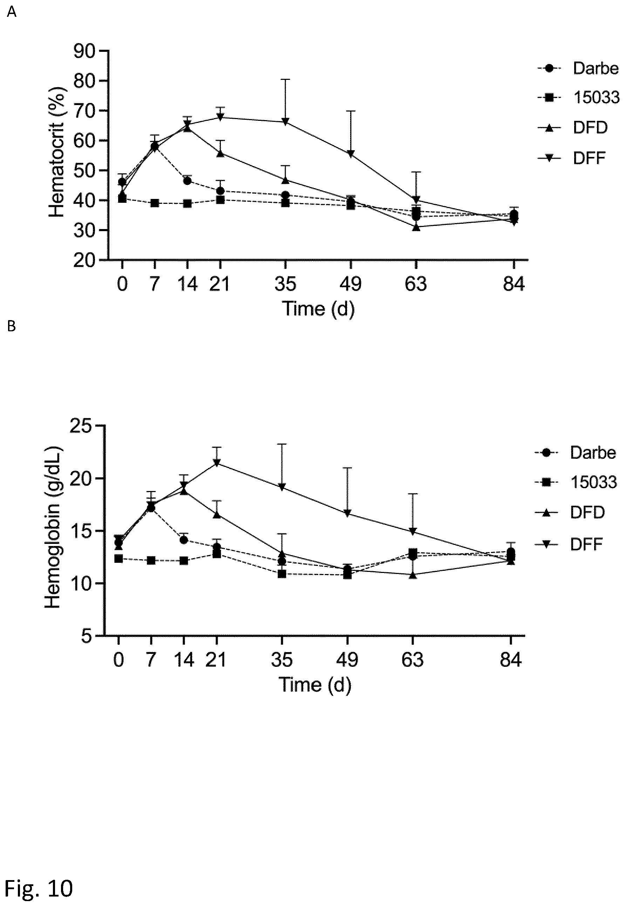

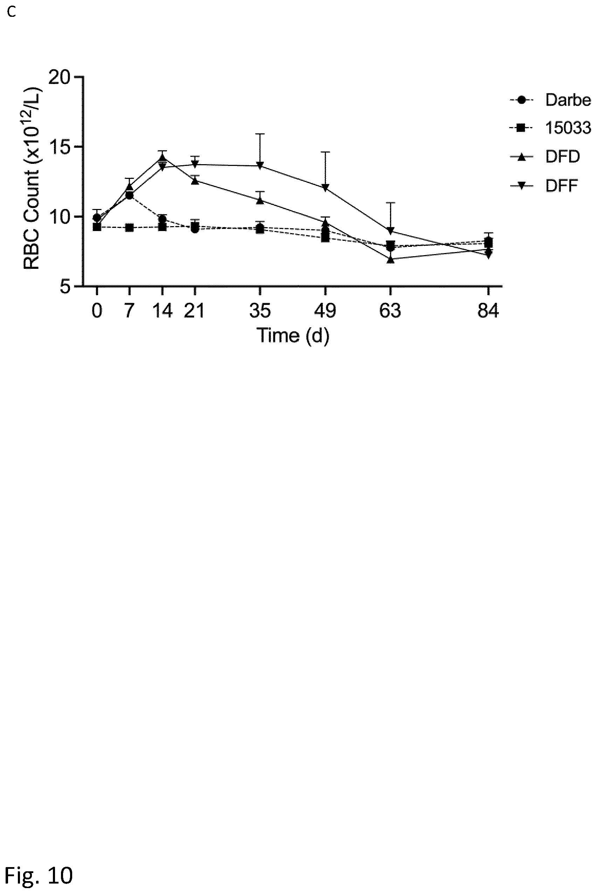

**FIGS. 10A to 10C** show *in vivo* mouse studies. **A.** Haematocrit measurements in humanized mice (mice with human *EPOR* knock-in) of the DFD and DFF molecules. **B.** Hemoglobin measurements in humanized mice of DFD and DFF molecules. **C.** Red Blood Cell (RBC) counts over time. Darbepoetin alfa (Darbe) and 15033 were used as positive and negative control, respectively.

### DETAILED DESCRIPTION OF THE INVENTION

In this disclosure, a few terms and abbreviations are used. The following definitions of such terms and abbreviations are provided.

As used herein, a person skilled in the relevant art may generally understand the term "comprising" to generally mean the presence of the stated features, integers, steps, or components as referred to in the claims, but that it does not preclude the presence or addition of one or more other features, integers, steps, components, or groups thereof.

As used herein, a person skilled in the relevant art can generally understand the term "erythropoietin" or its abbreviation "EPO" refers to the protein erythropoietin or when used in reference to a nucleic acid, the nucleic acid encoding EPO. As used herein, a person skilled in the relevant art will generally understand the term "erythropoietin receptor" or its abbreviation "EPOR" to refer to the EPO receptor or when used in reference to a nucleic acid, the nucleic acid encoding EPOR.

As used herein, a person skilled in the relevant art may generally understand the term "treatment" to generally refer to an approach for obtaining beneficial or desired results. Beneficial or desired results can include, but are not limited to, prevention or prophylaxis, alleviation, or amelioration of one or more symptoms or conditions, diminishment of the extent of a disease, stabilized (i.e., not worsening) state of disease, preventing spread of disease, delay or slowing of disease progression, amelioration or palliation of the disease state, and remission (whether partial or total), whether detectable or undetectable. "Treatment" can also mean prolonging survival as compared to expected survival if not receiving treatment.

As used herein, a person skilled in the relevant art may generally understand the term "therapeutically effective amount" to be an amount sufficient to effect treatment when administered to a subject in need of treatment. In the case of the embodiments of the present invention, a therapeutically effective amount can include, but is not limited to, an amount that eliminates or reduces the effects of the disease, such as for example, the tumour burden, in a subject.

As used herein, a person skilled in the relevant art may generally understand the term "amino acid sequence" to refer to an amino acid sequence of a naturally or non-naturally occurring protein molecule, "amino acid sequence" and like terms, such as "polypeptide" or "protein", are not meant to limit the amino acid sequence to the complete, native amino acid sequence associated with the recited protein molecule. Amino acid sequences can be referred to as having an amino (N) terminus and a carboxyl I terminus. Individual amino acids in a peptide or polypeptide can be referred to as "residues" and such residues are numbered sequentially beginning from the N-terminus and increasing towards the C-terminus. The amino acids located generally proximal to the N-terminus are generally referred to as the N-terminal amino acids while those located generally proximal to the C-terminus are referred to as the C-terminus amino acids. It will be understood by a person skilled in the relevant art that the reference to amino acid residues as either N terminus or C- terminus amino acid residues may vary depending on the protein.

As used herein, a person skilled in the relevant art may generally understand the terms "nucleic acid molecule encoding", "DNA sequence encoding," "RNA sequence encoding," "mRNA sequence encoding," "an oligonucleotide having a nucleotide sequence encoding a gene" "polynucleotide having a nucleotide sequence encoding a gene," "DNA encoding", "RNA encoding" and similar terminology to generally refer to the order or sequence of nucleotides along a single or double strand of nucleic acid comprising the coding region of a gene or, in other words, the nucleic acid sequence that encodes a gene product. The order of these nucleotides determines the order of amino acids along the polypeptide chain. The coding region may be present in a cDNA, genomic DNA, or RNA form. The oligonucleotide or polynucleotide may be single-stranded (e.g. the sense strand) or double-stranded (e.g. antisense and sense strands). Suitable control elements such as enhancers/promoters, splice junctions, polyadenylation signals, etc. may be placed near the coding region of the gene if needed to permit proper initiation of transcription and/or correct processing of the primary RNA transcript. Alternatively, the coding region utilized in expression vectors may contain endogenous enhancers/promoters, splice junctions, intervening sequences, polyadenylation signals, etc. or a combination of both endogenous and exogenous control elements.

A person skilled in the relevant art will understand that nucleic acid molecules are said to have "5' ends" and "3' ends" because mononucleotides are linked via a phosphodiester linkage to make oligonucleotides or polynucleotides in a manner such that the 5' phosphate of one mononucleotide pentose ring is attached to the 3' oxygen of its neighbour in one direction. Therefore, an end of an oligonucleotides or polynucleotide, referred to as the "5' end" if the 5' phosphate is not linked to the 3' oxygen of a preceding mononucleotide pentose ring and as the "3' end" if its 3' oxygen is not linked to the 5' phosphate of a subsequent mononucleotide pentose ring. As used herein, a nucleic acid sequence, even if internal to a larger oligonucleotide or polynucleotide, also may be said to have 5' and 3' ends. In either a linear or circular nucleic acid molecule, discrete elements are referred to as being "upstream" or 5' of the "downstream" or 3' elements. As a DNA molecule is typically provided in a double helix, the DNA molecule is said to have a "sense" strand and an "antisense" strand. The sense strand and the antisense strand are said to be reverse complementary in that the 3' end of the sense strand may anneal to the 5' end of the antisense strand and the 5' end of the antisense strand may anneal to the 3' end of the sense strand. The "sense" strand of the DNA molecule is typically copied into a messenger RNA (mRNA) during transcription. The mRNA made during transcription thus has the same sequence as the sense strand through transcription of the antisense strand so that the eventual protein may be based on the sense version of the DNA molecule. The term "antisense strand" is used in reference to a nucleic acid strand that is complementary to the "sense" strand. The designation (-) (i.e., "negative") is sometimes used in reference to the antisense strand, with the designation (+) (i.e., "positive") is sometimes used in reference to the sense.

As used herein, the term "homology" refers to a degree of complementarity. There may be partial homology or complete homology (i.e., identity). As applied to polypeptides, the term "substantial homology" as used herein means that two peptide sequences, when optimally aligned, such as by the programs GAP or BESTFIT using default gap weights, share at least 80 percent sequence identity, preferably at least 90 percent sequence identity, more preferably at least 95 percent sequence identity or more (e.g., 99 percent sequence identity). Amino acid sequences may differ by conservative amino acid substitutions. A person skilled in the relevant art will understand the term "conservative amino acid substitutions" to refer to the general interchangeability of residues having chemically similar side chains. For example, a group of amino acids having aliphatic side chains may comprise glycine, alanine, valine, leucine, and isoleucine; a group of amino acids having aliphatic-hydroxyl side chains may comprise serine and threonine; a group of amino acids having amide-containing side chains may comprise asparagine and glutamine; a group of amino acids having aromatic side chains may comprise phenylalanine, tyrosine, and tryptophan; a group of amino acids having basic side chains may comprise lysine, arginine, and histidine; and a group of amino acids having sulphur-containing side chains may comprise cysteine and methionine.

The term "fragment" as used herein in reference to single chain amino acid sequences refers to a polypeptide that may have an amino (N) terminus portion and/or carboxy (C) terminus portion deleted as compared to the native protein, but wherein the remaining amino acid sequence of the fragment is identical to the amino acid sequence of the native protein. It will be understood by a person skilled in the relevant art that the term "fragment" may also refer to a portion of a multi-chain protein molecule (e.g., antibody fragment)

The term "naturally occurring" or "native" as used herein as applied to an object refers to the fact that an object can be found in nature. For example, a polypeptide or polynucleotide sequence that is present in an organism (including viruses) that can be isolated from a source in nature, and which has not been modified is naturally occurring. A person skilled in the relevant art would understand that the term "synthetic" references a non-naturally occurring compound.

As used herein, the term "target," refers to a structure, such as, for example, a nucleic acid or protein molecule, to be identified, detected, characterized, or amplified. Thus, the "target" is sought to be sorted out from other structures.

The term "isolated" when used in relation to a nucleic acid or peptide, as in "an isolated oligonucleotide", "isolated polynucleotide" or "isolated polypeptide", refers to a nucleic acid or amino acid sequence that is identified and separated from at least one contaminant with which it is ordinarily associated in its natural source. Isolated compounds are present in a form or setting that is different from that in which it is found nature. In contrast, non-isolated compounds, such as nucleic acids or amino acid sequences, are found in the state they exist in nature. For example, a given DNA sequence (e.g., a gene) is found on the host cell chromosome in proximity to neighbouring genes; RNA sequences, such as a specific mRNA sequence encoding a specific protein, are found in the cell as a mixture with numerous other mRNAs that encode a multitude of proteins.

As used herein, the term "portion" when in reference to a nucleotide sequence or an amino acid sequence refers to fragments of that sequence.

As used herein, the term "purified" or "to purify" refers to the removal of contaminants from a sample. For example, EPOR agonists are purified by removal of contaminating non-immunoglobulin proteins; they are also purified by the removal of immunoglobulin that does not bind EPOR. The removal of non-immunoglobulin proteins and/or the removal of immunoglobulins that do not bind EPOR results in an increase in the percent of EPOR agonist in the sample.

The term "recombinant protein" or "recombinant polypeptide" as used herein refers to a protein molecule that is expressed from a recombinant DNA molecule.

Numerous techniques that are well known in the art are used to detect antibody binding in association with the present invention. These techniques include, but not limited to RIA (radioimmunoassay), ELISA (enzyme-linked immunosorbant assays), "sandwich" immunoassays, immunoradiometric assays, gel diffusion precipitation reactions, immunodiffusion assays, SEC (size exclusion chromatography), BLI (Biolayer Interferometry), in situ immunoassays (e.g. using colloidal gold, enzyme or radioisotope labels, for example), Western blots, precipitation reactions, agglutination assays (e.g. gel agglutination assays, hemagglutination assays, etc.), complement fixation assays, immunofluorescence assays, protein A assays, and immunoelectrophoresis assays, etc.

The term "antigenic determinant" as used herein refers to that portion of an antigen that contacts an antibody (i.e., an epitope). When a protein or fragment of a protein is used to immunize a host animal, numerous regions of the protein may induce the production of antibodies that bind specifically to a given region or three-dimensional structure on the protein; these regions or structures are referred to as antigenic determinants. An antigenic determinant may compete with the intact antigen (i.e., the "immunogen" used to elicit the immune response) for binding to an antibody. A person skilled in the art will understand that the "paratope", also referred to as the antigen-binding site, is the part of an antibody which recognizes and binds to the epitope of the antigen. Each paratope is made up of six complementarity-determining regions - three from each of the light and heavy chains. Each arm of the Y-shaped antibody has an identical paratope at the end.

The term "sample" as used herein is used in its broadest sense. A sample suspected of containing a nucleic acid or amino acid sequence may comprise a cell, chromosomes isolated from a cell (e.g., a spread of metaphase chromosomes), genomic DNA (in solution or bound to a solid support), RNA (in solution or bound to a solid support), cDNA (in solution or bound to a solid support) and the like. A sample suspected of containing a protein may comprise a cell, a portion of a tissue, an extract containing one or more proteins and the like.

As used herein, the term "response," refers to the generation of a detectable signal when used in reference to an assay or other result (e.g., accumulation of reporter molecule, increase in ion concentration, accumulation of a detectable chemical product (e.g., antibody)).

As used herein, the terms "agonist" and "agonistic" refer to or describe a molecule which is capable of, directly or indirectly, initiating, activating, stimulating, or inducing one of more aspects of a response (e.g., a physiological response) or other biological activity when combined with a receptor. In a preferred embodiment, the compounds of the present invention, such as diabodies, may comprise agonists of EPO in that they mimic one or properties of EPO. As used herein, the terms "antagonist" and "antagonistic" refer to or describe a molecule which is capable of, directly or indirectly, acting against or blocking one of more aspects a physiological response or other biological activity when combined with a receptor. An antagonist is the opposite of agonist. In another preferred embodiment of the present invention, the compounds of the present invention, such as complete antibodies, may comprise antagonists of EPO in that they inhibit the actions of EPO.

As used herein, the term "antibody" or "Ab" is used in the broadest sense and specifically covers single anti-EPOR monoclonal antibodies (including agonist, antagonist, and neutralizing or blocking antibodies) and anti-EPOR antibody compositions with polyepitopic specificity. "Antibody" as used herein includes intact immunoglobulin or antibody molecules, polyclonal antibodies, multispecific antibodies (i.e., bispecific antibodies formed from at least two intact antibodies), immunoglobulin or antibody fragments (such as Fab, F(ab')2, or Fv) and synthetic diabodies, so long as they exhibit any of the desired agonistic or antagonistic properties described herein. Various procedures known within the art may be used to produce such antibodies directed against a specific antigen, or against derivatives, fragments, analogues, homologs or orthologs thereof.

Antibodies are typically proteins or polypeptides that exhibit binding specificity to a specific antigen. Native antibodies are usually heterotetrameric glycoproteins, composed of two identical light (L) chains and two identical heavy (H) chains. Typically, each light chain is linked to a heavy chain by one covalent disulphide bond, while the number of disulphide linkages varies between the heavy chains of different immunoglobulin isotypes. Each heavy and light chain also has regularly spaced intrachain disulphide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains. The light chains of antibodies from any vertebrate species can be assigned to one of two clearly distinct types, called kappa and lambda, based on the amino acid sequences of their constant domains. Depending on the amino acid sequence of the constant domain of their heavy chains, immunoglobulins can be assigned to different classes. There are five major classes of immunoglobulins: IgA, IgD, IgE, IgG and IgM, and several of these may be further divided into subclasses (isotypes), e.g., IgG-1, IgG-2, IgG-3, and IgG-4; IgA-1 and IgA-2. The heavy chain constant domains that correspond to the different classes of immunoglobulins are called alpha, delta, epsilon, gamma, and mu, respectively.

As used herein, "antibody fragments" comprise a portion of an intact antibody, generally the antigen binding or variable region of the intact antibody. Examples of antibody fragments include Fab, Fab', F(ab')2, and Fv fragments, diabodies, single chain antibody molecules, and multispecific antibodies formed from antibody fragments.

The term "diabody" refers to small antibody fragments with two antigen-binding sites, wherein each antigen binding site comprises a heavy chain variable domain (VH) connected to a light chain variable domain (VL) in the same polypeptide chain (VH and VL). In a diabody, each VL domain is connected with one VH domain in a single-chain Fv (scFv) fragment by a short linker. By using a linker that is short (e.g., in a preferred embodiment of the present invention, 5 to 15 amino acids or 18Å) to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

As used herein, the term "linker" refers to a linking sequence that promotes the formation of a diabody, preferrable an 18Å (±2Å) amino acid linker, more preferably a linker having any amino acid combination of 5 to 15 amino acids, yet more preferably having the sequences GGGGG, IKGGGGGEV, LKVLSRGVV, ISARAGSLV, SKSRAGGEV, LKGGRGGKV, NKGGGGAKV, IKGSSRDDI, MKHAGRGGV, SKGGGGGEV, MKHAGRGGV, LECSDCSGI, yet even more preferably having the sequence GGGGG.

As used herein, the term "variable domain" describes certain portions of antibodies that differ in sequence among antibodies and are used in the binding and specificity of each antibody for its particular antigen. However, the variability is not usually evenly distributed through the variable domains of antibodies. It is typically concentrated in three segments called complementarity determining regions ("CDRs"), "hypervariable regions" or "hypervariable domains" both in the light chain and the heavy chain variable domains. The more highly conserved portions of the variable domains are called the framework regions ("FR"). The variable domains of native heavy and light chains each comprise four FR regions, largely adopting a beta-sheet configuration, connected by three CDRs, which form loops connecting, and in some cases forming part of, the beta-sheet structure. The CDRs in each chain are held together in proximity by the FR regions and, with the CDRs from the other chain, contribute to the formation of the antigen binding site of antibodies. The constant domains are not involved directly in binding an antibody to an antigen, but exhibit various effector functions, such as participation of the antibody in antibody-dependent cellular toxicity.

The term "Fc region" (the term "Fc domain" may be used interchangeably herein) is used to define a C-terminal region of an immunoglobulin heavy chain, including native-sequence Fc regions and variant Fc regions. The boundaries of the Fc region of an immunoglobulin heavy chain might vary; in some embodiments, the Fc region may include one or more amino acids of the hinge region. Suitable native- sequence Fc regions for use in the antibodies of the present disclosure include human IgG1, IgG2 (IgG2A, IgG2B), IgG3 and IgG4.

As used herein, the term "monoclonal antibody" or "mAb" refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen.

The modifier "monoclonal" indicates the character of the antibody as being obtained from a substantially homogeneous population of antibodies and is not to be construed as requiring production of the antibody by any method.

An "antibody construct" as provided herein refers to a recombinant polypeptide or recombinant polypeptide complex, which includes an antibody peptide (e.g., a first antibody peptide) including an antibody variable domain (e.g., first antibody variable domain) and an antibody domain. In embodiments, the antibody construct is an antibody, as described herein. In embodiments, the antibody construct is an antibody variant, as described herein. The antibody construct is capable of binding an antigen at least in part through said antibody variable domain and may be a monovalent or multivalent (e.g., divalent or tetravalent) polypeptide complex. The antibody construct provided herein may include one or more antibody variable domains (e.g., a first, second, third or fourth antibody variable domain) forming part of one or more antibody peptides (e.g., a first antibody peptide or a second antibody peptide). Therefore, the antibody construct may be able to bind an antigen at least in part through one or more (e.g., 2, 3, or 4) antibody variable domains and may therefore be referred to as a monovalent, a divalent or a tetravalent antibody construct, respectively.

The antibody constructs provided herein including embodiments thereof may be tetravalent antibody constructs. Where the antibody construct provided herein including embodiments thereof is a tetravalent antibody construct, the antibody construct includes four antigen-binding sites.

As used herein, a "tetravalent" antibody construct refers to an antibody that comprises four antibody VH-VL binding units, with each VH-VL binding unit comprising an antibody VH domain and an antibody VL domain.

In one embodiment, a first VL domain is linked at its carboxy terminus to a first VH domain (VL-VH orientation). In one embodiment, a first VH domain is linked at its carboxy terminus to a first VL domain (VH-VL orientation).

In one embodiment, all of the four antibody binding units are in the VL-VH orientation.

In one embodiment, all of the four antibody binding units are in the VH-VL orientation.

The antibody construct as provided herein includes at least one antibody peptide. An "antibody peptide" as provided herein is a polypeptide including a combination of an antibody variable domain provided herein with one or more heavy chain constant domains (e.g., CH₁, CH₂, or CH₃) or fragments or variants thereof. A "heavy chain constant (CH) domain" as provided herein is a domain (e.g., heavy chain constant 1 (CH₁) domain, heavy chain constant 2 (CH₂) domain, heavy chain constant 3 (CH₃) domain), which forms part of a constant region of a heavy chain of an antibody or a fragment thereof.

As described above the antibody construct may include more than one antibody peptide. In embodiments, the antibody construct includes a first antibody peptide and a second antibody peptide bound to each other. The first antibody peptide may be bound to the second antibody peptide through a chemical linker. The first antibody peptide may be bound to the second antibody peptide through a peptide linker. The first antibody peptide may be bound to the second antibody peptide through a covalent bond. The first antibody peptide may be bound to the second antibody peptide through a non-covalent bond. The first antibody peptide may be bound to the second antibody peptide to form a dimer.

An "antibody domain" as referred to herein is a portion of a polypeptide including a heavy chain constant region (CH). An antibody domain may include one or more heavy chain constant domains (e.g., CH₁, CH₂, and CH₃). In embodiments, the antibody domain, is the Fc domain, comprising a constant heavy chain 2 (CH₂) domain and a constant heavy chain 3 (CH₃) domain.

The antibody constructs provided herein are recombinant polypeptides that may include parts or fragments of an antibody (e.g., CDRs, VL, VH, CH₁, CH₂, CH₃ domains). In embodiments, the antibody construct is an antibody. In embodiments, the antibody construct is an antibody variant.

It is here claimed a tetravalent antibody construct that specifically binds to hEPOR, the tetravalent antibody construct comprising: (i) four light chain variable (VL) domains, wherein each of the four VL domains comprises a CDR-L1, a CDR-L2, and a CDR-L3; (ii) four heavy chain variable (VH) domains, wherein each of the four VH domains comprises a CDR-H1, a CDR-H2, and a CDR-H3; (iii) a Fc domain, wherein each of the four VL domains forms a VH-VL binding unit with a corresponding VH of the four VH domains and wherein each of the four VH-VL binding units is specific for hEPOR.

In some embodiment, the four VL domains comprises different CDR-L1, CDR-L2 and CDR-L3. In some embodiment, the four VL domains comprises identical CDR-L1, CR-L2 and CDR-L3.

In some embodiment, the four VH domains comprises different CDR-H1, CDR-H2 and CDR-H3. In some embodiment, the four VL domains comprises identical CDR-H1, CDR-H2 and CDR-H3.

In some embodiments, the antibody Fc domain is a human antibody Fc domain. In some embodiments, the antibody Fc domain is the human IgG1 Fc domain, having the sequence






 (SEQ ID NO: 19)

In some embodiment, the Fc domain is a portion of the IgG1 Fc.

In some embodiments, the Fc domain comprises one or more amino acid substitutions to reduce immune effector functions. In an embodiment, it comprises a proline to serine substitution at amino acid 331, numbering according to EU index. In an embodiment, it comprises the so-called 'LALA' double mutation (Leu234Ala together with Leu235Ala), numbering according to EU index.

In one embodiment, said four antigen-binding sites are provided by two diabodies, each one comprising two light chain variable domains VL and two heavy chain variable domains VH.

In one embodiment, said four antigen-binding sites are provided by a diabody, comprising a VL and a VH, and by two Fab, each one comprising one constant and one variable domain of each of the heavy and the light chain of a second antibody.

In one embodiment, said four antigen-binding sites are provided by four Fab.

In one embodiment, the diabody is bound to a Fc. In one embodiment, the diabody is bound to the CH₂ domain of said Fc through one or two peptide linkers. In one embodiment, the diabody is bound to the CH₃ domain of said Fc through one or two peptide linkers.

In one embodiment, in the antibody constructs comprising two diabodies, each one of them is bound to the CH₂ domain of the Fc via a peptide linker. In one embodiment, in the antibody constructs comprising two diabodies, one diabody is bound to the CH₂ domain of the Fc via two peptide linkers, one diabody is bound to the CH₃ domain of the Fc via two peptide linkers.

In one embodiment, the diabody is bound to two Fab via one peptide linker, one at the C terminus, the other at the N terminus. In one embodiment, a Fab is linked to a Fc via one or two peptide linkers.

In one embodiment, said tetravalent antibody construct comprises two Fab, each one of them linked via one peptide linker to a Fc domain.

In one embodiment, said tetravalent antibody construct comprises four Fab, each one of them linked via one peptide linker to a Fc domain. In one embodiment, two of said Fab bind the Fc at the CH₂ domain, two Fab bind the Fc at the CH₃ domain.

In one embodiment, when two linkers are present, the two linkers are connected to each other via disulfide linkers.

In one embodiment, each diabody-Fc is non-covalently connected to another diabody.

In one embodiment, a Fc domain binds a first diabody, via the CH₂ domain and a second diabody, via the CH₃ domain.

In one embodiment, a Fc domain binds a diabody via the CH₂ domain and a Fab via the CH₃ domain.

In one embodiment, an IgG binds a diabody via the CH₃ domain.

In one embodiment, a Fc domain binds a diabody via the CH₂ domain and said diabody binds a Fab.

In one embodiment, an IgG binds a Fab. In one embodiment, via the constant domains. In one embodiment, via the variable domains.

The antibody constructs provided herein are recombinant polypeptides that may include parts or fragments of an antibody (e.g., CDRs, FRs, VL, VH, CH₁, CH₂, CH₃ domains). In embodiments, the antibody construct is an antibody. In embodiments, the antibody construct is an antibody variant.

In embodiments, the antibody construct is a Fab' fragment. In embodiments, the antibody construct is a chimeric antibody. In embodiments, the antibody construct is a humanized antibody. In embodiments, the antibody construct is a human antibody.

In an embodiment, said antibody comprises a Fc and two diabodies, wherein said Fc binds via a first CH₂ domain a first diabody and via a second CH₂ domain a second diabody.

In an embodiment, said antibody comprises a Fc and two diabodies, wherein said Fc binds via the two CH₂ domains a first diabody and via the two CH₃ domain a second diabody.

In an embodiment, said antibody comprises a Fc, a diabody and a Fab, wherein said Fc binds via the two CH₂ domains the diabody and via the two CH₃ domain the Fab, or wherein said Fc binds via the two CH₃ domain the diabody and via the two CH₂ domain the Fab, or wherein said Fc binds via the two CH₂ domain the diabody and said diabody in turn binds the Fab.

In an embodiment, said antibody comprises an IgG and a Fab, wherein said IgG binds via the two CH₃ domains the Fab, or wherein said IgG binds via the two variable domains the Fab.

In one embodiment, said diabody comprises a CDR-L1 having a contiguous amino acid sequence SVSSA (SEQ ID NO: 1), a CDR-L2 having a contiguous amino acid sequence SASSLYS (SEQ ID NO: 2), a CDR-L3 having a contiguous amino acid sequence SSXFLI (SEQ ID NO: 3) and a CDR-H1 having a contiguous amino acid sequence LDSYYM (SEQ ID NO: 4), a CDR-H2 having a contiguous amino acid sequence SIAPYHGYTY (SEQ ID NO: 5), a CDR-H3 having a contiguous amino acid sequence HGYGAL (SEQ ID NO: 6).

In one embodiment, said Fab comprises a CDR-L1 having a contiguous amino acid sequence SVSSA (SEQ ID NO: 1), a CDR-L2 having a contiguous amino acid sequence SASSLYS (SEQ ID NO: 2), a CDR-L3 having a contiguous amino acid sequence SSXFLI (SEQ ID NO: 3) and a CDR-H1 having a contiguous amino acid sequence LDSYYM (SEQ ID NO: 4), a CDR-H2 having a contiguous amino acid sequence SIAPYHGYTY (SEQ ID NO: 5), a CDR-H3 having a contiguous amino acid sequence HGYGAL (SEQ ID NO: 6).

According to yet another embodiment of the present invention, there is provided the diabodies of the present invention with a linker that promotes the formation of a diabody, preferrable an 18Å (±2Å) amino acid linker, more preferably a linker having any amino acid combination of 5 to 15 amino acids, yet more preferably having the sequences GGGGG (SEQ ID NO: 7), IKGGGGGEV (SEQ ID NO: 8), LKVLSRGW (SEQ ID NO: 9), ISARAGSLV (SEQ ID NO: 10), SKSRAGGEV (SEQ ID NO: 11), LKGGRGGKV (SEQ ID NO: 12), NKGGGGAKV (SEQ ID NO: 13), IKGSSRDDI (SEQ ID NO: 14), MKHAGRGGV (SEQ ID NO: 15), SKGGGGGEV (SEQ ID NO: 16), MKHAGRGGV (SEQ ID NO: 17), LECSDCSGI (SEQ ID NO: 18), yet more preferably having the sequence GGGGG (SEQ ID NO: 7).

In one embodiment, the free VL-VH chain has the sequence

DIQMTQSPSSLSASVGDRVTITCRASQSVSSAVAWYQQKPGKAPKLLIYSASSLY
SGVPSRFSGSRSGTDFTLTISSLQPEDFATYYCQQSSNFLITFGQGTKVEIKEVQLVES
GGGLVQPGGSLRLSCAASGFTLDSYYMHWVRQAPGKGLEWVASIAPYHGYTYYADS
VKGRFTISADTSKNTAYLQMNSLRAEDTAVYYCARHGYGALDYWGQGTLVTVSS
(SEQ ID NO: 20)

In one embodiment, the VL-VH region, or the VH-VL region, are separated by a 1-15 amino acid linker.

In one embodiment, the VL and VH in the free-VL-VH region are linked via a Glycine (G1). The VL-VH G1 has the sequence:

DIQMTQSPSSLSASVGDRVTITCRASQSVSSAVAWYQQKPGKAPKLLIYSASSLY
SGVPSRFSGSRSGTDFTLTISSLQPEDFATYYCQQSSNFLITFGQGTKVEIKGEVQLVE
SGGGLVQPGGSLRLSCAASGFTLDSYYMHWVRQAPGKGLEWVASIAPYHGYTYYAD
SVKGRFTISADTSKNTAYLQMNSLRAEDTAVYYCARHGYGALDYWGQGTLVTVSS
(SEQ ID NO: 21).

In one embodiment, the VL and VH in the free-VL-CH region are linked via two Glycine (G2). The VL-VH G1 has the sequence:

DIQMTQSPSSLSASVGDRVTITCRASQSVSSAVAWYQQKPGKAPKLLIYSASSLY
SGVPSRFSGSRSGTDFTLTISSLQPEDFATYYCQQSSNFLITFGQGTKVEIKGGEVQLV
ESGGGLVQPGGSLRLSCAASGFTLDSYYMHWVRQAPGKGLEWVASIAPYHGYTYYA
DSVKGRFTISADTSKNTAYLQMNSLRAEDTAVYYCARHGYGALDYWGQGTLVTVSS
(SEQ ID NO: 22).

As an example, the free VL-VH region is comprised in the tetravalent antibody construct named D₂F, depicted in **FIG.** 2A.

In one embodiment, the two polypeptide chains comprised in the tetravalent antibody construct have the sequence

DIQMTQSPSSLSASVGDRVTITCRASQSVSSAVAWYQQKPGKAPKLLIYSASSLY
SGVPSRFSGSRSGTDFTLTISSLQPEDFATYYCQQSSNFLITFGQGTKVEIKEVQLVES
GGGLVQPGGSLRLSCAASGFTLDSYYMHWVRQAPGKGLEWVASIAPYHGYTYYADS
VKGRFTISADTSKNTAYLQMNSLRAEDTAVYYCARHGYGALDYWGQGTLVTVSSGGG
GSGGGGSEPKSSDKTHTCPPCPAPEAAGGPSVFLFPPKPKDTLMISRTPEVTCVVVD
VSHEDPEVKFNWYVDGVEVHNAKTKPREEQYNSTYRVVSVLTVLHQDWLNGKEYKC
KVSNKALPASIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALHNHY
TQKSLSLSPGK (SEQ ID NO: 23).

In one embodiment, the two polypeptide chains comprised in the tetravalent antibody construct have the sequence

DIQMTQSPSSLSASVGDRVTITCRASQSVSSAVAWYQQKPGKAPKLLIYSASSLY
SGVPSRFSGSRSGTDFTLTISSLQPEDFATYYCQQSSNFLITFGQGTKVEIKGEVQLVE
SGGGLVQPGGSLRLSCAASGFTLDSYYMHWVRQAPGKGLEWVASIAPYHGYTYYAD
SVKGRFTISADTSKNTAYLQMNSLRAEDTAVYYCARHGYGALDYWGQGTLVTVSSGG
GGSGGGGSEPKSSDKTHTCPPCPAPEAAGGPSVFLFPPKPKDTLMISRTPEVTCVVV
DVSHEDPEVKFNWYVDGVEVHNAKTKPREEQYNSTYRVVSVLTVLHQDWLNGKEYK
CKVSNKALPASIEKTISKAKGQPREPQVYTLPPSREEMTKNQVSLTCLVKGFYPSDIAV

EWESNGQPENNYKTTPPVLDSDGSFFLYSKLTVDKSRWQQGNVFSCSVMHEALHNH
YTQKSLSLSPGK (SEQ ID NO: 24).

In one embodiment, the two polypeptide chains comprised in the tetravalent antibody construct have the sequence

DIQMTQSPSSLSASVGDRVTITCRASQSVSSAVAWYQQKPGKAPKLLIYSASSLY
SGVPSRFSGSRSGTDFTLTISSLQPEDFATYYCQQSSNFLITFGQGTKVEIKGGEVQLV
ESGGGLVQPGGSLRLSCAASGFTLDSYYMHWVRQAPGKGLEWVASIAPYHGYTYYA
DSVKGRFTISADTSKNTAYLQMNSLRAEDTAVYYCARHGYGALDYWGQGTLVTVSSG
GGGSGGGGSEPKSSDKTHTCPPCPAPEAAGGPSVFLFPPKPKDTLMISRTPEVTCVV
VDVSHEDPEVKFNWYVDGVEVHNAKTKPREEQYNSTYRVVSVLTVLHQDWLNGKEY
KCKVSNKALPASIEKTISKAKGQPREPQVYTLPPSREEMTKNQVSLTCLVKGFYPSDIA
VEWESNGQPENNYKTTPPVLDSDGSFFLYSKLTVDKSRWQQGNVFSCSVMHEALHN
HYTQKSLSLSPGK (SEQ ID NO: 25).

In one embodiment, the tetravalent antibody construct comprises two diabody and an Fc. In one embodiment, the construct comprises two polypeptide chains having the sequence

DIQMTQSPSSLSASVGDRVTITCRASQSVSSAVAWYQQKPGKAPKLLIYSASSLY
SGVPSRFSGSRSGTDFTLTISSLQPEDFATYYCQQSSQFLITFGQGTKVEIKGGGGGE
VQLVESGGGLVQPGGSLRLSCAASGFTLDSYYMHWVRQAPGKGLEWVASIAPYHGY
TYYADSVKGRFTISADTSKNTAYLQMNSLRAEDTAVYYCARHGYGALDYWGQGTLVT
VSSGGGGSGGGGSEPKSSDKTHTCPPCPAPEAAGGPSVFLFPPKPKDTLMISRTPEV
TCVVVDVSHEDPEVKFNWYVDGVEVHNAKTKPREEQYNSTYRVVSVLTVLHQDWLN
GKEYKCKVSNKALPASIEKTISKAKGQPREPQVYTLPPSREEMTKNQVSLTCLVKGFY
PSDIAVEWESNGQPENNYKTTPPVLDSDGSFFLYSKLTVDKSRWQQGNVFSCSVMHE
ALHNHYTQKSLSLSPGKGGGGSGGGGSGGGGSGGGGSDIQMTQSPSSLSASVGDR
VTITCRASQSVSSAVAWYQQKPGKAPKLLIYSASSLYSGVPSRFSGSRSGTDFTLTISS
LQPEDFATYYCQQSSQFLITFGQGTKVEIKGGGGGEVQLVESGGGLVQPGGSLRLSC
AASGFTLDSYYMHWVRQAPGKGLEWVASIAPYHGYTYYADSVKGRFTISADTSKNTA
YLQMNSLRAEDTAVYYCARHGYGALDYWGQGTLVTVSS (SEQ ID NO: 26).

In one embodiment, the tetravalent antibody construct comprises a diabody and a Fab. In one embodiment, the construct comprises a heavy chain sequence 
DIQMTQSPSSLSASVGDRVTITCRASQSVSSAVAWYQQKPGKAPKLLIYSASSLY
SGVPSRFSGSRSGTDFTLTISSLQPEDFATYYCQQSSQFLITFGQGTKVEIKGGGGGE
VQLVESGGGLVQPGGSLRLSCAASGFTLDSYYMHWVRQAPGKGLEWVASIAPYHGY
 
TYYADSVKGRFTISADTSKNTAYLQMNSLRAEDTAVYYCARHGYGALDYWGQGTLVT
VSSGGGGSGGGGSEPKSSDKTHTCPPCPAPEAAGGPSVFLFPPKPKDTLMISRTPEV
TCVVVDVSHEDPEVKFNWYVDGVEVHNAKTKPREEQYNSTYRVVSVLTVLHQDWLN
GKEYKCKVSNKALPASIEKTISKAKGQPREPQVYTLPPSREEMTKNQVSLTCLVKGFY
PSDIAVEWESNGQPENNYKTTPPVLDSDGSFFLYSKLTVDKSRWQQGNVFSCSVMHE
ALHNHYTQKSLSLSPGKGGGGSGGGGSGGGGSGGGGSEVQLVESGGGLVQPGGSL
RLSCAASGFTLDSYYMHWVRQAPGKGLEWVASIAPYHGYTYYADSVKGRFTISADTS
KNTAYLQMNSLRAEDTAVYYCARHGYGALDYWGQGTLVTVSSASTKGPSVFPLAPSS
KSTSGGTAALGCLVKDYFPEPVTVSWNSGALTSGVHTFPAVLQSSGLYSLSSVVTVPS
SSLGTQTYICNVNHKPSNTKVDKKVEPKSCDKTHT (SEQ ID NO: 27).
 and a light chain sequence

DIQMTQSPSSLSASVGDRVTITCRASQSVSSAVAWYQQKPGKAPKLLIYSASSLY
SGVPSRFSGSRSGTDFTLTISSLQPEDFATYYCQQSSQFLITFGQGTKVEIKRTVAAPS
VFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC (SEQ ID NO: 28).

In one embodiment, the tetravalent antibody construct comprises a IgG and a diabody, as an example it is the IgG-diabody construct depicted in FIG. 5A. In one embodiment, the construct comprises a heavy chain sequence 
EVQLVESGGGLVQPGGSLRLSCAASGFTLDSYYMHWVRQAPGKGLEWVASIAP
YHGYTYYADSVKGRFTISADTSKNTAYLQMNSLRAEDTAVYYCARHGYALDYWGQG
TLVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPASIEKTISKAKG
QPREPQVYTLPPSREEMTKNQVSLTCLVKGFYPSDIAVEWESNGQPENNYKTTPPVL
DSDGSFFLYSKLTVDKSRWQQGNVFSCSVMHEALHNHYTQKSLSLSPGKGGGGSGG
GGSGGGGSGGGGSDIQMTQSPSSLSASVGDRVTITCRASQSVSSAVAWYQQKPGKA
PKLLIYSASSLYSGVPSRFSGSRSGTDFTLTISSLQPEDFATYYCQQSSQFLITFGQGTK
VEIKGGGGGEVQLVESGGGLVQPGGSLRLSCAASGFTLDSYYMHWVRQAPGKGLEW
VASIAPYHGYTYYADSVKGRFTISADTSKNTAYLQMNSLRAEDTAVYYCARHGYALD
YWGQGTLVTVSS (SEQ ID NO: 29).
 and a light chain sequence of SEQ ID NO: 28.

In one embodiment, the tetravalent antibody construct comprises a Fab, a diabody and a Fc, as an example it is the FDF construct depicted in **FIG.** 6A. In one embodiment, the construct comprises a heavy chain sequence 
EVQLVESGGGLVQPGGSLRLSCAASGFTLDSYYMHWVRQAPGKGLEWVASIAP
YHGYTYYADSVKGRFTISADTSKNTAYLQMNSLRAEDTAVYYCARHGYGALDYWGQG
TLVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTG
GGGSGGGGSGGGGSGGGGSDIQMTQSPSSLSASVGDRVTITCRASQSVSSAVAWY
QQKPGKAPKLLIYSASSLYSGVPSRFSGSRSGTDFTLTISSLQPEDFATYYCQQSSQFL
ITFGQGTKVEIKGGGGGEVQLVESGGGLVQPGGSLRLSCAASGFTLDSYYMHWVRQ
APGKGLEWVASIAPYHGYTYYADSVKGRFTISADTSKNTAYLQMNSLRAEDTAVYYCA
RHGYGALDYWGQGTLVTVSSGGGGSGGGGSEPKSSDKTHTCPPCPAPEAAGGPSV
FLFPPKPKDTLMISRTPEVTCVVVDVSHEDPEVKFNWYVDGVEVHNAKTKPREEQYN
STYRVVSVLTVLHQDWLNGKEYKCKVSNKALPASIEKTISKAKGQPREPQVYTLPPSR
EEMTKNQVSLTCLVKGFYPSDIAVEWESNGQPENNYKTTPPVLDSDGSFFLYSKLTVD
KSRWQQGNVFSCSVMHEALHNHYTQKSLSLSPGK (SEQ ID NO: 30)
 and a light chain sequence of SEQ ID NO: 28.

In one embodiment, the tetravalent antibody construct comprises a IgG and a Fab, as an example it is the IgG-Fab construct depicted in FIG. 7A. In one embodiment, the construct comprises a heavy chain sequence 
EVQLVESGGGLVQPGGSLRLSCAASGFTLDSYYMHWVRQAPGKGLEWVASIAP
YHGYTYYADSVKGRFTISADTSKNTAYLQMNSLRAEDTAVYYCARHGYGALDYWGQG
TLVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPASIEKTISKAKG
QPREPQVYTLPPSREEMTKNQVSLTCLVKGFYPSDIAVEWESNGQPENNYKTTPPVL
DSDGSFFLYSKLTVDKSRWQQGNVFSCSVMHEALHNHYTQKSLSLSPGKGGGGSGG
GGSGGGGSGGGGSEVQLVESGGGLVQPGGSLRLSCAASGFTLDSYYMHWVRQAPG
KGLEWVASIAPYHGYTYYADSVKGRFTISADTSKNTAYLQMNSLRAEDTAVYYCARHG
YGALDYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVDKKV
EPKSCDKTHT (SEQ ID NO: 31)
 and a light chain sequence SEQ ID NO: 28.

In one embodiment, the tetravalent antibody construct comprises a IgG and a Fab, as an example it is the Fab-IgG construct depicted in **FIG. 8A****.** In one embodiment, the construct comprises a heavy chain sequence 
EVQLVESGGGLVQPGGSLRLSCAASGFTLDSYYMHWVRQAPGKGLEWVASIAP
YHGYTYYADSVKGRFTISADTSKNTAYLQMNSLRAEDTAVYYCARHGYGALDYWGQG
TLVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTG
GGGSGGGGSGGGGSGGGGSEVQLVESGGGLVQPGGSLRLSCAASGFTLDSYYMH
WVRQAPGKGLEWVASIAPYHGYTYYADSVKGRFTISADTSKNTAYLQMNSLRAEDTA
VYYCARHGYGALDYWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNVNHKPS
NTKVDKKVEPKSCDKTHTCPPCPAPEAAGGPSVFLFPPKPKDTLMISRTPEVTCVVVD
VSHEDPEVKFNWYVDGVEVHNAKTKPREEQYNSTYRVVSVLTVLHQDWLNGKEYKC
KVSNKALPASIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALHNHY
TQKSLSLSPGK (SEQ ID NO: 32)
 and a light chain sequence SEQ ID NO: 28.

It will be understood by a person skilled in the relevant art that modifications of the antibodies of the present invention are contemplated herein. The antibodies of the present invention may be modified by conjugating, tagging or labelling through methods known in the art, the antibodies of the present invention to any known diagnostic or therapeutic agent, including but not limited to cytotoxic agents (e.g., immunotoxin conjugates), prodrugs, drugs (e.g., pharmaceutically active substances) or other effector molecules which are effective in the treatment of disease as well as known reporter molecules. Such modified antibodies, also referred to as immunochemical derivatives thereof include, but are not limited to (a) labelled (e.g. radiolabelled, enzyme-labelled, fluorochrome or chemiluminescent compound) monoclonal antibodies of the present invention, preferably humanized mAbs, for diagnosing or detecting tumours and tumour spread (e.g. metastasis) using known imaging technologies; and (b) immunotoxin conjugates of the mAbs of the present invention, preferably humanized mAbs, where the mAbs of the present invention are conjugated to known cytotoxic, radioactive, radiolabelled, prodrug or drug moieties (e.g. radioimmunotherapy). It will be understood by a person skilled in the relevant art that the term "cytotoxic agent", "cytotoxins" or "cytotoxic" as used herein generally refer to a substance that inhibits or prevents the function of cells and/or causes destruction of cells and includes, but is not limited to, radioactive isotopes, chemotherapeutic agents, and toxins such as small molecule toxins or enzymatically active toxins of bacterial, fungal, plant or animal origin, including fragments and/or variants thereof. It will also be understood by a person skilled in the relevant art that the term "prodrug" as used in this application generally refers to a precursor or derivative form of a pharmaceutically active substance that is less cytotoxic to target cells compared to the pharmaceutically active substance and is capable of being activated or converted into the more pharmaceutically active substance.

It may be understood by a person skilled in the relevant art that antibodies of the present invention can also be made by recombinant DNA methods, such as those described in U.S. Pat. No. 4,816,567, which is hereby incorporated by reference. DNA encoding the monoclonal antibodies of the invention can be readily isolated and sequenced using conventional procedures (e.g., by using oligonucleotide probes that are capable of binding specifically to genes encoding the heavy and light chains of murine antibodies). Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy and light chain constant domains in place of the homologous murine sequences (U.S. Pat. No. 4,816,567; Morrison, Nature 368, 812-13 (1994)) or by covalently joining to the immunoglobulin coding sequence all or part of the coding sequence for a non-immunoglobulin polypeptide. Such a non-immunoglobulin polypeptide can be substituted for the constant domains of an antibody of the invention or can be substituted for the variable domains of one antigen-combining site of an antibody of the invention to create a chimeric bivalent antibody.

It may be understood by a person skilled in the art that "phage display" references technology for generating and selecting novel proteins that bind to a ligand, such as an antigen. Using the technique of phage display, large libraries of protein variants can be generated and rapidly sorted for those sequences that bind to a target antigen with high affinity. Methods of generating peptide libraries and screening those libraries have been disclosed in many patents (e.g., U.S. Pat. No. 5,723,286, U.S. Pat. No. 5,432,018, U.S. Pat. No. 5,580,717, U.S. Pat. No. 5,427,908 and U.S. Pat. No. 5,498,530). It will also be understood that antibody phage display libraries may also be created. (Smith et al., Science (1985), 228:1315; Skerra and Pluckthun, Science (1988), 240:1038). Libraries of antibodies or antigen binding polypeptides have been prepared in a number of ways including by altering a single gene by inserting random DNA sequences or by cloning a family of related genes. Methods for displaying antibodies or antigen binding fragments using phage display have been described in U.S. Pat. Nos. 5,750,373, 5,733,743, 5,837,242, 5,969,108, 6,172,197, 5,580,717, and 5,658,727. The antibody phage display library is then screened for expression of antibodies or antigen binding proteins with desired characteristics. Phage display technology has several advantages over conventional hybridoma and recombinant methods for preparing antibodies with the desired characteristics. This technology allows the development of large libraries of antibodies with diverse sequences in less time and without the use of animals. Preparation of hybridomas or preparation of humanized antibodies can easily require several months of preparation. In addition, since no immunization is required, phage antibody libraries can be generated for antigens which are toxic or have low antigenicity (Hogenboom, Immunotechniques (1988), 4:1-20). Commercially available, such as those developed by Cambridge Antibody Technology and Morphosys (Vaughan et al. (1996) Nature Biotech 14:309; Knappik et al. (1999) J. Mol. Biol. 296:57).

It will be understood by a person skilled in the relevant art that the compositions of the present invention, including but not limited to synthetic tetravalent antibody constructs, can be formulated into pharmaceutical compositions for administration in a manner customary for administration of such materials using standard pharmaceutical formulation chemistries and methodologies, all of which are readily available to a person skilled in the relevant art. It will also be understood by a person skilled in the relevant art that such pharmaceutical compositions may include one or more excipients, carriers, stabilizers, or other pharmaceutically inactive compounds, such as, but not limited to, wetting or emulsifying agents, pH buffering substances and the like. Pharmaceutically acceptable salts can also be included therein. A thorough discussion of pharmaceutically acceptable excipients, vehicles and auxiliary substances is available in Remington's Pharmaceutical Sciences (Mack Pub. Co. N.J. 1991). Such pharmaceutical compositions can be prepared as injectable or oral preparations. The antibody constructs of the present invention may be administered by injection, including, but not limited to, intramuscular, intravenous, subcutaneous, peritoneal, transdermic or nasal injection. The therapeutically effective doses may vary according to body weight and the timing and duration of administration will be determined by specific clinical research protocols.

The description that follows, and the embodiments described therein, are provided by way of illustration of an example, or examples, of particular embodiments of the principles and aspects of the present invention. These examples are provided for the purposes of explanation, and not of limitation, of those principles and of the invention. In the description, like parts are marked throughout the specification and the drawings with the same respective reference numerals.

The present invention is directed to the diagnosis and treatment of key disease or cancer-associated anaemia without adverse effects on disease (e.g., cancer, kidney disease) recurrence and patient survival through the selective activation or signalling of the EPOR. More preferably, embodiments of the present invention are directed to specific synthetic binding molecules, including antibodies, diabodies, etc. as a treatment for disease, including cancer associated anaemia. More preferably, the present invention is directed to methods of activating an EPOR activation site upon binding with the agonists of the present invention. The invention may be applicable to many disease states, including but not limited to cancer associated anaemia and kidney disease alone or in combination with other therapeutic agents.

EPOR agonists demonstrated excellent function in promoting erythropoiesis *in vitro* and long-lasting activity *in vivo.* These EPOR agonists may represent a novel therapeutic option for patients with chronic kidney disease or treatment related anaemia.

### EXAMPLES

### In vitro functional activity of EPOR Binding Molecules

Activation of EPOR triggers the proliferation of human erythroid cell lines. Thus, the ability of various antibody constructs and species to stimulate proliferation of the human erythroid UT7/EPO cell line was tested.

Unlike a typical IgG, the diabody-Fc (DF) molecule lacks the CH₁ and CL domains **(****FIG. 1A****).** Instead, the VL and VH domains are connected via a short linker of 5-15 amino acids and fused directly to the CH₂ domain of the Fc. Following transient expression in Chinese hamster ovarian (CHO) cells and protein-A capture, the DF sample was further purified using size exclusion chromatography (SEC) to isolate the major species. The results, in **FIG. 1B****,** show that the protein-A captured material is a mixture consisting of 3 peaks that corresponded to 3 different species of varying molecular weights - a monomer, which is the major peak (Peak 3, DF) as well as two smaller peaks (Peaks 1, D₃F₃ and 2, D₂F₂) that are trimeric and dimeric non-covalently associated species, respectively. Using a UT7/EPO cell proliferation assay, Peak 3, which is the major DF monomeric species, had the lowest activity **(****FIG. 1C****).** Peaks 1 and 2, which correspond to the molecular weights of the trimer and dimer, respectively, possessed much higher activity. 15033 is a bivalent DF molecule that recognizes a viral antigen and serves as a suitable negative control in all cellular and *in vivo* assays. This data indicates that valency is a critical determinant of functional agonism and that the major monomeric species for the DF modality is suboptimal for EPOR activation.

The non-covalent DF dimer is highly active and should function as a tetravalent (FIG. 1A to 1C). Thus, several tetravalent antibody constructs were developed and tested, all of which share the same VL and VH domain, wherein said VL comprises a CDR-L1 having a contiguous amino acid sequence SVSSA (SEQ ID NO: 1), a CDR-L2 having a contiguous amino acid sequence SASSLYS (SEQ ID NO: 2), a CDR-L3 having a contiguous amino acid sequence SSQFLI (SEQ ID NO: 3) and said VH comprises a CDR-H1 having a contiguous amino acid sequence LDSYYM (SEQ ID NO: 4), a CDR-H2 having a contiguous amino acid sequence SIAPYHGYTY (SEQ ID NO: 5), a CDR-H3 having a contiguous amino acid sequence HGYGAL (SEQ ID NO: 6).

The tetravalent D₂F construct, schematics of the embodiments are depicted in **FIG. 2A****,** is similar to the DF except that instead of two VL-VH-Fc chains covalently binding to each other, two additional, separate free VL-VH chains are non-covalently binding to form the D₂F molecule. The D₂F molecule can adopt a configuration schematically shown in FIG. 2A (left panel) or other tetravalent configurations including a tetrabody-Fc with all 4 VL-VH regions forming a lattice (right panel). Varying Glycine linker lengths between the VL and the VH domains in both the free as well as the DF chains, as shown in **FIG. 2B****,** were tested using the UT7/EPO cell proliferation assay **(****FIG. 2C****).**

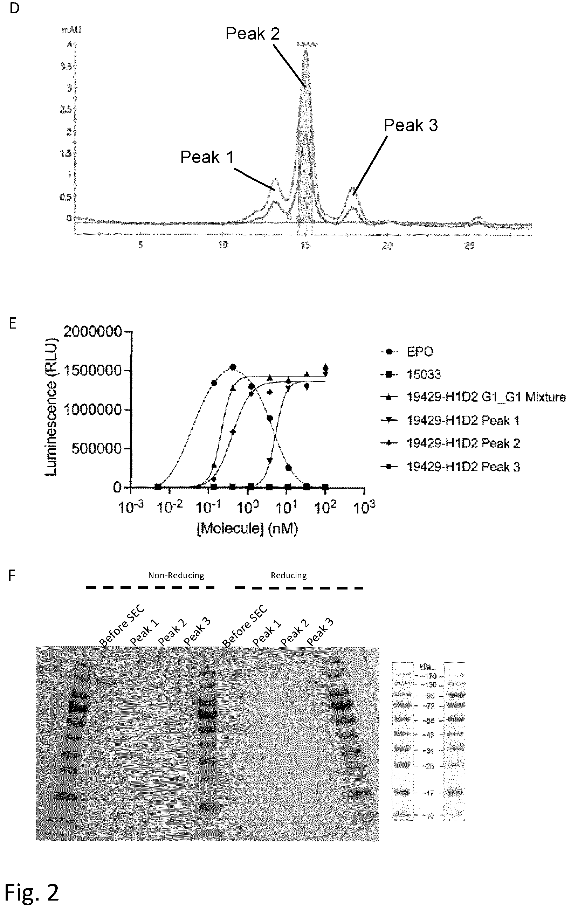

Following protein-A affinity purification, all constructs had robust activity **(****FIG. 2C****).** However, the G1_G1 D₂F variant, i.e. the variant wherein the VL and the VH are linked by a single Glycine in both the free and DF chains, had the least number of aggregated species as shown by SEC **(****FIG. 2D****).** The biological agonistic activity of these peaks was tested using a UT7/EPO cell proliferation assay **(****FIG. 2E****).** Unlike the DF construct in which the major species was only marginally active as a monomer **(****FIG. 1C****),** the major D₂F Peak 2 exhibited the greatest agonism **(****FIG. 2E****).** SDS-PAGE analysis revealed that under non-reducing conditions, the free VL-VH chain (~25 kDa) co-eluted with the VL-VH-Fc chain (~105 kDa), consistent with a non-covalently associated dimer **(****FIG. 2F****).** Under reducing conditions, the VL-VH-Fc chain dissociates into two ~50 kDa chains while the free VL-VH chain is unaffected **(****FIG. 2F****).** These data validate the G1_G1 D₂F as a viable, EPOR agonistic tetravalent molecule.

While the D₂F tetravalent consists of four chains, a simpler modality is to covalently connect the free variable (VH-VL) chain diabody to the DF (in VH-VL format) chain via a short peptide linker to create a single-chain D₂F (scD₂F) **(****FIG. 3A****).** This molecule only has 2 chains. Much like the D₂F, the scD₂F can adopt tetravalent configurations as represented in FIG. 3A including association of the distal VH-VL with the proximal VH-VL (left panel) or a tetrabody-Fc where all variable chains associate as a lattice (right panel). Following protein-A purification, SEC analysis revealed that this scD₂F construct largely existed as a single main species with additional higher-molecular-weight species **(****FIG. 3B****).** This main peak was isolated and tested in a UT7/EPO cell proliferation assay to assess its erythropoietic activity. Shown in **FIG. 3C****,** this main peak robustly induced proliferation, suggesting that the D₂F modality is highly active.

The tetravalent DFD construct, **FIG. 4A****,** consist of VL-VH diabodies at each end of an Fc connected via peptide linkers. Following protein-A purification, 3 species were identified and purified via SEC **(****FIG. 4B****).** Each peak was then tested using a UT7/EPO cell proliferation assay to determine their agonistic properties **(****FIG. 4C****).** All peaks were highly active, including the major Peak 3, which corresponds to the DFD monomer.

The DFF construct shown in **FIG. 5A** is similar to the DF molecule except that it has two Fabs attached to the CH₃ domain of the Fc. The protein-A captured mixture was then subjected to purification using SEC. The results, in **FIG. 5B****,** show that the DFF construct is a mixture consisting of 4 peaks corresponding to 4 different species of varying molecular weights. Activity of all peaks was then tested with a UT7/EPO cell proliferation assay **(****FIG. 5C****).** While the major Peak 4 was not as active as the minor Peaks 1 to 3, it still robustly induced UT7/EPO cell proliferation. Also, it is still markedly more potent (i.e. lower EC50) and more efficacious (i.e. higher max activity) than the major DF monomer species **(****FIG. 1C****).** Thus, the DFF is a viable drug modality since its predominant form in solution can activate EPOR.

The IgG-Diabody is shown in **FIG. 6A** and consists of a VL-VH diabody covalently linked to the CH₃ domain at the C-terminus of an IgG. It predominantly exists as a single species **(****FIG. 6B****).** Given its homogeneity and its monodispersity, the IgG-Diabody mixture was tested for activity in a UT7/EPO cell proliferation assay. This analysis revealed that the IgG-Diabody tetravalent was potent and efficacious in erythropoietic activity **(****FIG. 6C****).**

The FDF construct is a DF molecule with Fabs connected at the N-terminus of the VL-VH domains **(****FIG. 7A****).** SEC analysis revealed that the protein-A captured FDF mixture consisted of 3 species/peaks **(****FIG. 7B****).** Functional testing revealed that the major peak 3 had significantly reduced activity compared to the original mixture as well as the minor peaks **(****FIG. 7C****).** This result is in stark contrast to the G1_G1, DFD, and DFF tetravalents in which the major peaks were all very active at inducing EPOR activity and highlights the novelty of our invention. Despite having identical paratopes (i.e. CDR sequences), the geometry of the paratopes is essential for biological activity. Not all geometries are suitable and those that work appreciably are novel and non-obvious.

The IgG-Fab molecule shown in **FIG. 8A** is an IgG with Fabs attached to the CH₃ domain of the Fc. SEC analysis found that the protein-A purified material only has 1 species **(****FIG. 8B****).** Functional analysis revealed that the IgG-Fab molecule only modestly induced UT7/EPO cell proliferation **(****FIG. 8C****).**

The Fab-IgG molecule shown in **FIG. 9A** is an IgG with Fabs attached to the VH domain via a peptide linker. Similar to the IgG-Fab, SEC analysis of the Fab-IgG revealed that it also exists largely as a single species following protein-A purification **(****FIG. 9B****).** The Fab-IgG molecule was only weakly active **(****FIG. 9C****)** in a UT7/EPO cell proliferation assay.

The arrangement of the VL and VH domains in the diabody portion of the tetravalents was also tested. The DFF molecule shown in **FIG. 4A** consists of one N-terminal diabody covalently attached onto each Fc. The VL-VH domain arrangement was flipped to VH-VL while all other structural features of the molecule remained unchanged. SEC analysis revealed that this Reverse DFF molecule predominantly existed as a single species. Assessment of its erythropoietic activity using a UT7/EPO cell proliferation assay revealed that it is active, too (data not shown). This data indicates that the orientation of the diabody - whether it is VL-VH or VH-VL - does not significantly impact its agonistic properties.

### In vivo functional activity of EPOR Binding Molecules

Based on activity using the UT7/EPO cell proliferation assay, the main monomeric species of the DFD (Peak 3, **FIG. 4B**) and DFF (Peak 4, **FIG. 5B**) molecules were tested in a humanized mouse model of erythropoiesis (Huang X et al. Erythropoietin receptor signalling regulates both erythropoiesis and megakaryopoiesis in vivo. Blood Cells, Molecules, and Diseases 2010; 44:1-6). The endogenous *EPOR* of these mice is replaced by the human *EPOR* in its proper genetic locus. At Day 0, 3 animals were injected with each monomeric molecule at a dose of 0.2 mg/kg or 0.1 mg/kg of darbepoietin (Darbe) as a positive control. The 15033 DF molecule was used as a negative control. At the days indicated, blood was drawn from these mice and haematological parameters including haematocrit, haemoglobin content, and red blood cell (RBC) count were measured (FIGS. 10A to C). The effects of darbepoietin in these mice are fairly transient, peaking at Day 7 and then returning to baseline by Day 21. This is consistent with the ~7 hr half-life of darbepoietin in rodents (Egrie C et al. Darbepoetin alfa has a longer circulating half-life and greater in vivo potency than recombinant human erythropoietin. Exp Hematol. 2003;31:290-9). In contrast, the effects of monomeric DFD and DFF persist much longer. In the case of DFD-injected mice, hematologic parameters peaked at Day 14 and remain elevated until past Day 35. The effects of DFF were even more dramatic. The DFF monomer induced a slow and gradual increase in haematocrit, haemoglobin content, and RBC numbers until peaking at Day 21 before slowly dropping over the course of the next 2 months back to baseline. These results indicate that both the DFD and DFF monomer species are highly active *in vivo* to activate the human EPOR. Their pro-erythropoietic effects persist far longer than darbepoietin, which is the current standard of care for anaemia in CKD patients.

## Claims

1. A tetravalent antibody construct comprising 4 VL domain and 4 VH domain, each one of said 4 VL domain comprising a CDR-L1 having a contiguous amino acid sequence SVSSA (SEQ ID NO: 1), a CDR-L2 having a contiguous amino acid sequence SASSLYS (SEQ ID NO: 2), a CDR-L3 having a contiguous amino acid sequence SSXFLI (SEQ ID NO: 3), each one of said 4 VH domain comprising a CDR-H1 having a contiguous amino acid sequence LDSYYM (SEQ ID NO: 4), a CDR-H2 having a contiguous amino acid sequence SIAPYHGYTY (SEQ ID NO: 5), a CDR-H3 having a contiguous amino acid sequence HGYGAL (SEQ ID NO: 6).

2. The construct according to claim 1, wherein in said SEQ ID NO: 3 X is Q or X is N.

3. The construct according to claim 1, wherein said 4 VL share a complete homology among them.

4. The construct according to claim 1, wherein said 4 VH domain share a complete homology among them.

5. The construct according to claim 1, consisting in a tetrabody.

6. The construct according to claim 1, comprising two diabodies.

7. The construct according to claim 1, comprising a diabody and two Fab.

8. The construct according to claim 1, comprising four Fab.

9. The construct according to claim 1, further comprising a Fc domain, comprising a constant heavy chain 2 (CH₂) domain and a constant heavy chain 3 (CH₃) domain.

10. The construct according to claim 9, wherein said Fc domain is IgG1-Fc.

11. The construct according to claim 10, wherein said Fc domain comprises one or more amino acid substitutions.

12. The construct according to claim 11, wherein said substitutions consist in a proline to serine substitution at amino acid 331, numbering according to EU index, a Leu 234 to Ala and a Leu 235 to Ala, wherein the numbering is according to EU index.

13. The construct according to any one of the claims 1-12 comprising one diabody, wherein said diabody is linked to the CH₂ domain of said Fc or to the CH₃ domain of said Fc through one or two peptide linkers.

14. The construct according to one of the claims 1-12 comprising two diabodies, wherein one diabody is bound to the CH₃ domain of said Fc, one diabody is bound to the CH₂ domain of said Fc through one or two peptide linkers.

15. The construct according to one of the claims 1-12 comprising a diabody and two Fab, wherein said diabody is linked to said Fab though one or two peptide linkers.

16. The construct according to one of the claims 1-12 comprising two Fab, each one of them linked via one peptide linker to said Fc domain.

17. The construct according to one of the claims 1-12 comprising four Fab, each one of them linked via one or two peptide linkers to said Fc domain.

18. The construct according to one of the claims 1-12 comprising 4 VL-VH region separated by 1-15 amino acid linker.

19. The construct according to one of the claims 1-12 comprising 4 VH-VL region separated by 1-15 amino acid linker.

20. A polynucleotide encoding a tetravalent antibody construct of any one of claims 1 to 19.

21. A vector comprising a polynucleotide of claim 20.

22. A host cell transfected with a vector of claim 21.

23. The tetravalent antibody construct of claim 1 for use in the treatment of kidney disease and anaemias, preferably in the treatment of chronic kidney disease, treatment-related anaemia, and inherited erythropoietin deficiency.

24. The tetravalent antibody construct for the use according to claim 22, wherein the target cell antigen is EPOR.
